# EUROPEAN PATENT APPLICATION

(11) **EP 2 947 156 A1**
(43) Date of publication of application: **25.11.2015**
(21) Application number: 14169524.7
(22) Date of filing: 22.05.2014
(51) Int. Cl.: C12Q 1/68

(54) **Optimization of sequencing reactions**

(71) Applicant: Qiagen GmbH, 40724 Hilden (DE)
(72) Inventor: Fang, Nan, 40724 Hilden (DE); Löffert, Dirk, Dr., 40724 Hilden (DE); Dhanapal, Vidya, Waltham, MA-02451 (DE); Werner, Martina, Dr., Winchester, MA 01890 (US); Kotler, Lev, Dr., Allston, MA 02134 (US); Mohan, Varuna, Allston, MA 02134 (US)
(74) Representative: Witte, Weller & Partner Patentanwälte mbB

(57) **Abstract**

The present invention relates to methods for sequencing of target nucleic acids, the method comprising the steps of (i) performing a clonal amplification step of target nucleic acids on a solid substrate to generate amplified amplification products coupled to the substrate,(ii) treating the reaction mixture containing the amplified amplification products coupled to the solid substrate from step (i) with an enzyme exhibiting single-strand-specific nuclease activity, and (iii) subjecting the amplified amplification products obtained after treatment in step (ii) to a sequencing-by-synthesis process thereby sequencing the target nucleic acid. Also, the present invention relates to the use of an enzyme exhibiting single-strand-specific nuclease activity in a, e.g. massively parallel, sequencing process, and to kits comprising such an enzyme.

SEQUENCE LISTING

<110>  QIAGEN GmbH

<120>  Optimization of Sequencing Reactions

<130>  4203P107EP

<160>  4

<170>  PatentIn version 3.5

<210>  1
<211>  45
<212>  DNA
<213>  Artificial Sequence

<220>
<223>  Forward Primer conjugated on beads


<220>
<221>  misc_feature
<222>  (1)..(1)
<223>  Bisbiotin

<400>  1
tttttttttt tttttttttt ttttttttgt aaaacgacgg ccagt                45


<210>  2
<211>  27
<212>  DNA
<213>  Artificial Sequence

<220>
<223>  Forward Primer


<220>
<221>  misc_feature
<222>  (1)..(1)
<223>  Bisbiotin

<400>  2
tttttttttt gtaaaacgac ggccagt                                    27


<210>  3
<211>  25
<212>  DNA
<213>  Artificial Sequence

<220>
<223>  Reverse Primer (B2)

<400>  3
acttcaattt actatgtagc aaagg                                      25


<210>  4
<211>  46
<212>  DNA

<213>  Artificial Sequence

<220>
<223>  Sequencing Primer

<400>  4
acttcaattt actatgtagc aaaggatact ccgacgcggc cgcagc                    46

## Description

The present invention is directed to novel methods for sequencing target nucleic acids, the method at least comprising the steps of performing a clonal amplification step of a target nucleic acid on a solid substrate to generate amplified PCR products coupled to the substrate, and the step of subjecting the amplified PCR products to a sequencing-by-synthesis process thereby sequencing the target nucleic acid.

### FIELD OF THE INVENTION

The present invention relates to the field of molecular biology, more particularly to sequencing processes including amplification reactions, and, in particular, to the optimization of the sequencing performance, e.g., in a massive parallel sequencing process.

### BACKGROUND OF THE INVENTION

The development of new sequencing instruments ("next generation" or "massively parallel") had a massive impact on genomics, since next generation sequencing (NGS) enables the generation of millions to hundreds of millions of reads in the same sequencing run. As a consequence, this technique has already found numerous applications in molecular and evolutionary biology, metagenomics, and clinical areas, such as in the analysis of genomes, in human genetics, forensics, prenatal screening, early detecting of cancer, etc.

Many NGS platforms differ in engineering configurations and sequencing chemistry. However, most sequencing approaches use an *in vitro* cloning step to amplify individual DNA molecules, because their molecular detection methods are not sensitive enough for single molecule sequencing. Thus, the recent sequencing platforms all share the technical paradigm of massive parallel sequencing via spatially separated, clonally amplified DNA templates or single DNA molecules in a flow cell. This design is very different from that of Sanger sequencing - also known as capillary sequencing or first-generation sequencing - which is based on electrophoretic separation of chain-termination products produced in individual sequencing reactions.

DNA sequencing with commercially available NGS platforms is - generally speaking - conducted with the following steps. First, DNA fragments are ligated with platform-specific sequencing adaptors to generate sequencing libraries. A PCR step generally follows the ligation of the sequencing adaptors to achieve sufficient amount of library molecules that can be used in the next steps. Secondly, DNA sequencing templates are generated by clonal amplification of the sequencing library molecules *in vitro* to generate thousands to hundreds of thousands of the identical copies from the same sequencing library molecule. Third, the spatially segregated, amplified DNA templates are sequenced simultaneously in a massively parallel fashion without the requirement for a physical separation step. The clonally amplified sequencing template can be sequenced either by synthesis (such as Illumina or Qiagen platforms) whereas the DNA sequence is determined by the addition of nucleotides to the complementary strand rather through chain-termination chemistry; or sequencing by ligation (such as LIFE Technologies Solid platform). While these steps are followed in most NGS platforms, each utilizes a different strategy.

Accordingly, one key element to most commercially available next generation sequencing technologies is the use of clonal amplification methods to prepare sequencing templates in a parallel, high-throughput manner from target nucleic acids, i.e. single DNA library fragments. The commonly used clonal amplification methods include Bridge Amplification (such as for the Illumina NGS platforms), emulsion PCR (such as QIAGEN GeneReader, Roche 454, Ion Torrent PGM and Proton platforms), and Wildfire (LIFE Technologies Solid).

All currently available clonal amplification methods use DNA polymerase and primers specific to the adaptor sequences of the sequencing library to generate identical copies of DNA from a single molecule in the sequencing library. One or both of the amplification primers used are bound to a solid surface so that the amplified DNA fragments can be concentrated and captured on this solid surface and subsequently sequenced.

In emulsion PCR methods, the sequencing library is subjected to PCR amplification in oil:water emulsions, where the majority of the aqueous droplets contain either 0 or 1 library molecule together with beads that are conjugated with PCR primers, and where, as a consequence, each of the droplets capturing one bead is a PCR microreactor that produces amplified copies of the single DNA template. Following the PCR reaction and washing steps, the thus generated double-stranded PCR products on the bead surfaces are denaturated to generate single-stranded sequencing templates. These sequencing templates attached to the surface of the beads can be hybridized with the platform-specific primers and subsequently sequenced.

With bridge amplification, forward and reverse primers are covalently attached at high-density to surface of a flow cell that allows bridge amplification of the fragments on its surface. The flow cell is exposed to reagents for polymerase-based extension, and priming occurs as the free end of a ligated fragment "bridges" to a complementary oligonucleotide on the slide surface. Repeated denaturation and extension results in localized amplification of DNA fragments in millions of separate locations across the flow cell surface. This solid-phase amplification produces millions spatially separated template clusters on the flow cell surface, providing free ends to which a universal sequencing primer is then hybridized to initiate the subsequent sequencing reaction.

Despite the advantages that massively parallel DNA sequencing has been provided so far, the sequencing accuracy still remains a major task to be dealt with. Due to the different template generation and preparation steps preceding the actual sequencing reaction, the whole process is prone to errors. PCR amplification of heterogeneous mixtures can result in population skewing, leading to over- or under-representation of particular variants. Also, polymerase mistakes, such as mis-incorporations and rearrangements, may occur during pre-amplification, as well as errors during clonal amplification, which may lead to an incorrect identification of bases.

Thus, it is an object of the present invention to provide for novel methods for massively parallel sequencing of target nucleic acids to overcome the drawbacks of the prior art and to provide for an improved method optimizing the sequencing performance in general.

### SUMMARY OF THE INVENTION

According to the invention, this object is solved by a method for sequencing of target nucleic acids, the method comprising the steps of (i) performing a clonal amplification step of target nucleic acids on a solid substrate to generate amplified amplification products coupled to the substrate in a reaction mixture, (ii) treating the reaction mixture containing the amplified amplification products coupled to the solid substrate from step (i) with an enzyme exhibiting single-strand-specific nuclease activity, and (iii) subjecting the amplified products obtained after treatment in step (ii) to a sequencing-by-synthesis process thereby sequencing the target nucleic acids.

The object underlying the invention is completed solved in that way.

With the method according to the invention, the accuracy of and overall sequencing performance has been greatly improved: Due to the enzyme exhibiting single-strand-specific nuclease activity which is employed after a clonal amplification step it is possible to eliminate single-stranded molecules on the solid substrate, which otherwise, after the clonal amplification step, are likely to impact the sequencing performance and lead to wrong sequencing results. By specifically employing a nuclease activity in the reaction mixture containing the amplification products as specified above after the clonal amplification step, a carry-over contamination with, e.g., single-stranded nucleic acid amplification products present on the solid substrate and/or of free PCR/amplification primers is efficiently prevented.

Presently, and as generally understood, a single-strand-specific nuclease is to be understood as an enzyme capable of specifically cleaving the phosphodiester bonds between the nucleotide subunits of single-stranded nucleic acids, in particular of single-stranded DNA or RNA molecules, whereby the enzyme does not digest or cleave double-stranded nucleic acids.

Nucleases comprise endonucleases and exonucleases, with some of the enzymes falling in both categories.

Thus, and according to one aspect of the invention, the single-strand-specific nuclease may be a single-strand specific exonuclease, in particular a single-strand-specific 3'->5' exonuclease, or a single-strand-specific endonuclease.

The step of treating amplification products with a single-strand-specific nuclease enzyme, e.g. with an enzyme exhibiting a 3'->5' exonuclease activity, can be easily incorporated in the protocol of a massively parallel sequencing or next generation sequencing as described at the outset, thereby improving the overall sequencing performance and accuracy of the process as such.

Thus, the present invention in particular refers to methods for massively parallel sequencing including the step of clonal amplification.

Presently, and as generally understood, the term "amplifying" or "amplification" in the context of nucleic acids refers to the production of multiple copies of a polynucleotide, or a portion of the nucleic acid molecule/polynucleotide, typically starting from a small amount of the polynucleotide (e.g., a single polynucleotide molecule), where the amplification products or "amplicons" are generally detectable. As such, a polymerase chain reaction represents one type of amplification reactions where a pair of primers is used that flank a desired target sequence. In conventional PCR the primers are mixed with a solution containing the target DNA (the template), a thermostable DNA polymerase and deoxynucleoside triphosphates (dNTPs). The reaction mixture is then heated to a temperature sufficient to separate the two complementary strands of the DNA template, and subsequently cooled to a temperature sufficient to allow the primers to specifically anneal to sequences flanking the gene or sequence of interest.

Accordingly, the expression "amplification reaction" as presently used is meant to designate a reaction amplifying a piece of DNA. In the case of PCR reaction, this consists of cycles of repeated heating and cooling of a reaction mixture for DNA melting and enzymatic replication of the DNA. Key components in a PCR amplification reaction are primers, i.e. short DNA fragments containing sequences complementary to a target region of the DNA, and a DNA polymerase, which allow for a selective and repeated amplification. As PCR amplification progresses, the DNA generated is itself used as a template for replication, setting in motion a chain reaction in which the DNA template is exponentially amplified.

Accordingly, an amplification reaction consists of a first round of a PCR reaction comprising several cycles of cooling and heating of a PCR reaction mixture. Typically, one PCR "cycle" consists of a series of between 20 to 80 repeated temperature changes, with each cycle commonly consisting of 2 to usually 3 discrete temperature steps. The cycling is often preceded by a single temperature step at a high temperature (>90°C), and followed by one hold at the end for final product extension or brief storage. The temperatures used and the length of time that are applied in each cycle depend on a variety of parameters, including the enzyme used for DNA synthesis, the concentration of divalent ions and dNTPs in the reaction, and the melting temperature (Tm) of the primers.

As used herein, the term "primer" refers to an oligonucleotide that is capable of acting as a point of initiation of synthesis when placed under conditions in which synthesis of a primer extension product that is complementary to a nucleic acid strand is induced (e.g., in the presence of nucleotides and a DNA polymerase or the like, and at a suitable temperature and pH).

As used herein, the term "nucleic acid molecule" refers to any nucleic acid containing molecule, including but not limited to, DNA or RNA. The term encompasses sequences that include any of the known base analogs of DNA and RNA including, but not limited to, 4 acetylcytosine, 8-hydroxy-N6-methyladenosine, aziridinylcytosine, pseudoisocytosine, 5- (carboxyhydroxyl-methyl) uracil, 5-fluorouracil, 5-bromouracil, 5-carboxymethylaminomethyl-2-thiouracil, 5-carboxymethyl-aminomethyluracil, dihydroura-cil, inosine, N6-isopentenyladenine, 1-methyladenine, 1-methylpseudo-uracil, 1- methyl-guanine, 1-methylinosine, 2,2-dimethyl-guanine, 2-methyladenine, 2-methylguanine, 3-methyl-cytosine, 5-methylcytosine, N6-methyladenine, 7-methylguanine, 5- methylaminomethyluracil, 5-methoxy-amino-methyl-2-thiouracil, beta-D-mannosylqueosine, 5'-methoxycarbonylmethyluracil, 5-methoxyuracil, 2-methylthio-N- isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As and when used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP). As is used herein, a nucleobase includes natural and modified residues, as described herein.

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. Presently, the expressions "primer" or "oligonucleotide" or "oligonucleotide primer" are used to designate an oligonucleotide functioning as a primer as defined above.

As used herein, the terms "complementary" or "complementarity" are used in reference to polynucleotides (i.e., a sequence of nucleotides) related by the base-pairing rules. For example, the sequence "5'-A-G-T-3', is complementary to the sequence "3 -T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "amplification product" is used herein to designate products, which are produced by the extension of a primer. The products are at least partially double stranded, for example, in the region comprising the primer extension product and its complement. Accordingly, "double stranded" or at least partially double-stranded amplification products can be generated with an amplification reaction.

The expression "clonal amplification" as used herein represents the amplification of a target nucleic acid sequence or a set of target nucleic acid sequence in a spatially or physically separated manner such that the amplified products are spatially or physically separated from the amplified products of other target sequences. E.g., bridge amplification (two-dimensional polymerase chain reaction), emulsion polymerase chain reaciton or rolling cycle amplification represent clonal amplification methods, which are presently used in the relevant field.

Thus, according to one aspect of the invention, the clonal amplification is selected from, but not limited to,bridge amplification, emulsion polymerase chain reaction (PCR), rolling circle amplification, helicase depending amplification (HDA), recombinase polymerase amplification, Loop-mediated isothermal amplification (LAMP), Strand displacement amplification (SDA), Multiple displacement amplification (MDA), Nicking enzyme amplification reaction (NEAR), Multiple Annealing and Looping Based Amplification Cycles (a.k.a MALBAC), SPIA (Single Primer Isothermal Amplification), WildFire Isothermal Amplification, and Avalanche from Ion Torrent/LIFE Technologies.

The above mentioned techniques are *perse* known in the relevant field and, as a consequence, known to one skilled in the art.

Accordingly, the amplification products generated in step (i) can be double stranded, as it is the case when employing, e.g., a polymerase chain reaction (PCR), or partially double stranded, as, e.g., in a rolling circle amplification (RCA).

As a consequence, and according to one aspect of the invention, the amplification products generated in step (i) are double-stranded, and the enzyme exhibiting single-strand specific nuclease activity employed in step (ii) can be an exonuclease or an endonuclease as defined above.

According to yet another aspect of the invention, the amplification products generated in step (i) are partially double-stranded, and the enzyme exhibiting single-strand specific nuclease activity employed in step (ii) is an exonuclease as defined above.

As such, in the method according to the invention, a set of target nucleic acid sequences are amplified which are attached to, bound to, or hybridized to a solid surface. Hereby, a "solid surface" represents any matrix being suitable to having attached thereto one or several nucleic acid molecules. A solid surface may have a wide variety of forms, including membranes, slides, plates, microchips, microparticles, beads, etc., and may have various shapes and features. As used herein, the terms "solid surface" and "solid substrate" are used interchangeably.

As such, a solid surface can be, e.g. isolated surfaces, a set of beads, or wells in a plate, such, that each surface on average contains one or fewer target nucleic acid sequences. The amplification is then carried out under conditions, such, that the amplified products of the target nucleic acid sequence of a respective surface are attached to, bound to, or hybridized to that surface. Generally, the respective surface comprises substantially no other amplified products from other target nucleic acid sequences.

A "sequencing-by-synthesis" (SBS) process refers to methods for determining the nucleotide sequence of a target nucleic acid by a polymerase extension reaction, whereby during the process one or more template nucleic acid strands, which may be provided in any suitable manner, are sequenced. Thus, contrary to the chain-termination chemistry used by the Sanger-technique, with the SBS process the nucleic acid molecules are sequenced by synthesis, determining the sequence by the addition of nucleotides to the complementary strand.

According to one aspect of the invention, the amplified template DNA strands may be coupled to or associated with a solid support, such as a microparticle, bead, or the like, and are loaded into reaction chambers. According to another aspect, the amplified template polynucleotide strands may be associated with a substrate surface or present in a liquid phase with or without being coupled to a support.

During a typical sequencing reaction, a primer anneals to a template nucleic acid single-strand to form a primer-template duplex, and is extended by a polymerase incorporating a nucleotide onto the 3' end of the primer. The primer-templatepolymerase complex is subjected to repeated exposures of different nucleotides. If a nucleotide(s) is incorporated, then the signal resulting from the incorporation reaction is detected. A wash step may be performed to remove unincorporated nucleotides prior to the next nucleotide exposure. After repeated cycles of nucleotide addition, primer extension, and signal acquisition, the nucleotide sequence of the template strand may be determined.

Within the methods according to the invention, any of various techniques for detecting the nucleotide incorporation(s) may be employed, such as, e.g. the detection of pyrophosphate (PPi) released by the incorporation reaction or the detection of labels associated with the nucleotides, such as mass tags, fluorescent, and/or chemiluminescent labels.

According to one aspect of the invention, the clonal amplification step in step (i) is bridge amplification or an emulsion polymerase chain reaction (PCR), and, accordingly, the solid substrate is selected from a flow cell or beads, respectively.

In emulsion PCR, clonal amplification is carried out by separating the target nucleic acid sequences and their amplified products from other target nucleic acid sequences and their amplified products, providing a set of microreactors, i.e. micro-droplets. Alternatively, the target nucleic acid sequences and their amplified products are separated from other target nucleic acids by providing them in a set of wells.

Also, in one aspect of the invention, in step (i) of the method according to the invention the clonal amplification step is an emulsion PCR to generate emulsion PCR beads having PCR products coupled thereto, that further in step (ii) the emulsion PCR beads from step (i) are treated with an enzyme exhibiting single-strand specific nuclease activity, and that the beads obtained after treatment in step (ii) are subjected to a sequencing-by-synthesis process.

Accordingly, the solid surface as used according to the invention can be a plurality of beads of any convenient size and fabricated from any number of known materials, such as natural and synthetic polymers, inorganics. A couple of examples are known to those skilled in the art, and are apparent from the present description. The beads are generally about 2 to about 100 µm in diameter, preferably 5 to about 80 pm in diameter, and more preferably about 10 to about 40 µm in diameter, and the beads may optionally be magnetic.

When employing emulsion PCR target nucleic acids are attached on beads included in an emulsion, thus functioning as a microreactor. The nucleic acids can be attached to the beads via a ligand attached to the nucleic acid to a receptor bound to the solid surface. As used herein, a microreactor is a small volume of fluid, generally in the volume range of microliters to nanoliters. In an emulsion PCR, a plurality of microreactors within the emulsion have one or fewer beads. The small volumes of the microreactors are isolated from one another allowing reactions to occur within each microreactor - and, thus, on the beads - without significant contamination from or mixing with other microreactors unless desired. Each droplet contains a heat-stable polymerase, dNTPs, buffer, adaptor specific PCR primers, a primer-conjugated bead and a library molecule. Following the PCR reaction and washing steps, the thus generated double-stranded PCR products on the bead surfaces are denaturated to generate single-stranded sequencing templates. These sequencing templates attached to the surface of the beads can be hybridized with the platform-specific primers and subsequently sequenced.

Generally, water-in-oil emulsions are used, which have small water droplets dispersed in a hydrophobic medium. As used herein, the term oil is used broadly to refer to a hydrophobic fluid in which aqueous droplets can be dispersed. The emulsion can be formed according to any suitable method known in the art.

According to one embodiment, in the method according to the invention, the enzyme exhibiting single-strand-specific nuclease activity is selected from at least one of the following: Exonuclease I (Exol) from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilago nuclease, and PfuExo I from *Pyrococcus furiosus.* A further overview and review discussing single-strand-specific nucleases that can be employed with the present invention can be found, e.g. in Desai NA and Shankar V., "Single-strand-specific nucleases", FEMS Microbiol Rev. 2003 Jan;26(5):457-91. Presently, with the expression "at least one" it is meant to employ or use one or a combination of two, three or more enzymes.

E.g., Exol is a 3'→5' single-strand-specific exonuclease that can digest single-stranded amplification primers. This enzyme is known in the art and is, e.g., commercially available from NEB or LIFE Technologies, Carlsbad, CA, USA.

In the method of the invention, according to one embodiment, step (ii) is performed for a time period of 5 min to 120 min, preferably for about 30 min to 70 min, and most preferably for about 30 min.

It is to be understood that the time periods above do not need to be exactly the ones as given, but that also deviances above and below the given ranges will allow the method to be performed equally good, and that the exact time will depend on the specific enzyme used, the nucleic acids and primers employed, as well as the temperature used for the incubation. The determination of suitable time periods will, thus, lie within the knowledge and skill of one skilled in the art upon reading of the present disclosure.

According to one embodiment, and in particular when using Exol, step (ii) is performed at about 37°C; as for the time period explained above, also for the temperature applied it will be apparent for one skilled in the art to use a temperature allowing the enzyme to properly react with the single-stranded nucleic acids. Accordingly, while 37°C will be appropriate for Exol, other temperatures for other enzymes exhibiting single-strand nuclease activity will be suitable or appropriate.

According to another embodiment, in the method of the invention after step (i) and prior to step (ii) the emulsions are broken and optionally washed.

According to this embodiment, following amplification of the nucleic acid template and the attachment of amplification copies to the bead, the emulsion is "broken". There are many methods of breaking an emulsion and one of skill in the art would be able to select an appropriate method, e.g. using an additional oil phase and/or adding an suitable organic solvent, such as alcohols, e.g. methanol, ethanol, and the like.

In yet another embodiment of the invention, in the method after step (ii) and prior to step (iii) the enzyme exhibiting single-strand-specific nuclease activity is heat in-activated.

Also, the method according to the invention may, in one embodiment, further comprise that after step (ii) and prior to step (iii) the amplified PCR products are denaturated to generate single-stranded sequencing templates on the substrate.

Denaturation of the amplified PCR products, which in one embodiment will represent double-stranded DNA PCR products, will be necessary to be able to perform the sequencing step. Thus, and according to one aspect of the invention, the amplified double-stranded nucleic acids templates attached to the solid surface needs to be single stranded. In such cases, the strand unattached to the solid surface may be melted away or "denaturated" using any number of commonly known methods such as, e.g., heat treatment, addition of NaOH, application of low ionic (e.g., salt) strength, enzymatic degradation, etc. Where the solid surface comprises a plurality of beads, following this strand removal step, the beads can be pelleted and the supernatant discarded. The beads can then be optionally washed and/or subsequently resuspended in a buffer, for example, in a sequencing reaction according to known technologies. After adding of a sequencing primer, this primer anneals to the single stranded amplification product, preferably using appropriate annealing conditions, such as annealing buffer and temperature conditions.

In yet another embodiment, in the method of the invention, prior to step (i), a library comprising or consisting of the target nucleic acid is prepared.

Typically, a conventional DNA library construction protocol consists of 4 steps, i.e., the fragmentation of the - generally larger- nucleic acid molecules, such as genomic DNA, the end repair of fragmented nucleic acids, e.g., DNA, the ligation of adapter sequences, and optional a library amplification.

Currently different methods are commonly used to generate fragmented nucleic acid molecules, e.g., fragmented genomic DNA, such as enzymatic digestion, sonication, nebulization, and hydrodynamic shearing. The resulting DNA fragment size distribution can be controlled by agarose gel electrophoresis or automated DNA analysis.

As examples, the nucleic acid molecules may be genomic DNA, cDNA, episomal DNA, BAC DNA, or YAC DNA. The genomic DNA may be animal, plant, viral, bacterial, or fungal genomic DNA. Preferably, the genomic DNA is human genomic DNA or human cDNA.

Following fragmentation, the nucleic acid sections must be repaired to generate blunt-ended, 5'-phosphorylated ends compatible with the sequencing platform-specific adapter ligation strategy.

Depending on the sequencing platform used, the blunt-ended (DNA) fragments can either directly be used for adapter-ligation, or need the addition of a single A overhang at the 3' ends of the DNA fragments to facilitate subsequent ligation of platform-specific adapters with compatible single T overhangs. T4 DNA ligase then adds the double-stranded adapters to the end-repaired library fragments, followed by reaction cleanup and DNA size selection to remove free library adapters and adapter dimers. The methods for size selection include agarose gel isolation, the use of magnetic beads, or advanced column-based purification methods.

After this step, DNA fragment libraries should be qualified and quantified. Depending on the concentration and adapter design of the sequencing library, it can either be directly diluted and used for sequencing, or subjected to optional library amplification. In the library amplification step, high-fidelity DNA polymerases are employed to either generate the entire adapter sequence needed for subsequent clonal amplification and binding of sequencing primers, with overlapping PCR primers, and/or to produce higher yields of the DNA libraries.

Thus, according to one embodiment of the method according to the invention, the method may comprise at least the following steps: fragmenting the nucleic acid template to be analyzed, e.g. genomic DNA, by any method known in the art, e.g. by shearing, nebulization, etc.; ligating adaptors to the ends of the fragments; capturing the individual fragments via the adaptors onto their respective own beads; mixing the beads and amplification reagents with an appropriate oil to prepare an emulsion, and performing a clonal amplification by means of PCR; breaking the emulsion and optionally washing the beads; reacting the beads with an enzyme exhibiting single-strand nuclease activity; inactivating the enzyme exhibiting single-strand nuclease activity; optionally washing the beads; denaturating newly synthesized double-strands to generate single-stranded sequencing templates on the bead surface; hybridizing with sequencing primers and performing a sequencing-by-synthesis. In a last step, the sequencing images are analyzed, thereby determining the sequences.

The invention also relates to the use of an enzyme exhibiting single-strand nuclease activity in massively parallel sequencing of a target nucleic acid, the sequencing method comprising a clonal amplification step and a sequencing-by-synthesis process.

The embodiments as described for the method according to the invention, and the advantages connected therewith, also do explicitly apply for the use according to the invention.

Accordingly, in one embodiment the use according to the invention it is preferred if the enzyme exhibiting single-strand nuclease activity is selected from an endonuclease or an exonuclease, preferably a 5'->3' single-strand exonuclease. More preferably, the enzyme exhibiting single-strand-specific nuclease activity is selected from at least one of the following: Exonuclease I (Exol) from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilago nuclease, and PfuExo I from *Pyrococcus furiosus.*

Also, the present invention relates to kits useful for carrying out the methods and uses of the invention.

In one aspect, the kit comprises (i) an enzyme exhibiting single-strand-specific nuclease activity, preferably an endonuclease or an exonuclease, and (ii) at least one of the following: a T4 ligase, Klenow fragment, amplification primer, one or more DNA polymerases, one or more adaptors, a solid surface, such as a bead or a set of beads, a substantially planar array, a well or wells in a plate, or an isolated surface, reagents for forming an emulsion buffer for one or more of the foregoing, sequencing primer. In some cases, the kit may further comprise instructions for the use of said kit. The components of the kits may comprise the same aspects and embodiments as described above for the components in the description of methods. For example, the ligands and receptors, the primers, the enzymes and the oligonucleotides can be those described herein to carry out the methods of the invention.

According to one aspect of the invention, the enzyme exhibiting single-strand-specific nuclease activity is selected from at least one of the following: Exonuclease I (Exol)from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilago nuclease, and PfuExo I from *Pyrococcus furiosu*

Further advantages follow from the description of the embodiments and the attached drawings.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description. In the figures:
- Fig. 1: shows schematic drawings of two hypotheses for suboptimal sequencing performances: **(A):** Primer-conjugated beads Inhibit SBS by competing for sequencing reagents; **(B):** Carry-over free amplification primer Inhibits SBS;
- Fig. 2: shows the sequencing images that were analyzed with Image J software (http://imagej.nih.gov/ij/). The signal intensity of the live emulsion PCR beads of the sample without Exol treatment **(A),** and of the sample with Exol treatment **(B);**
- Fig. 3: shows a diagram displaying the error rates in SBS (sequencing by synthesis) of Exol treated samples as compared to non-Exol treated samples; and
- Fig. 4: shows a diagram displaying mapped and perfect reads for Exol treated samples and non-Exol-treated samples.

### Examples

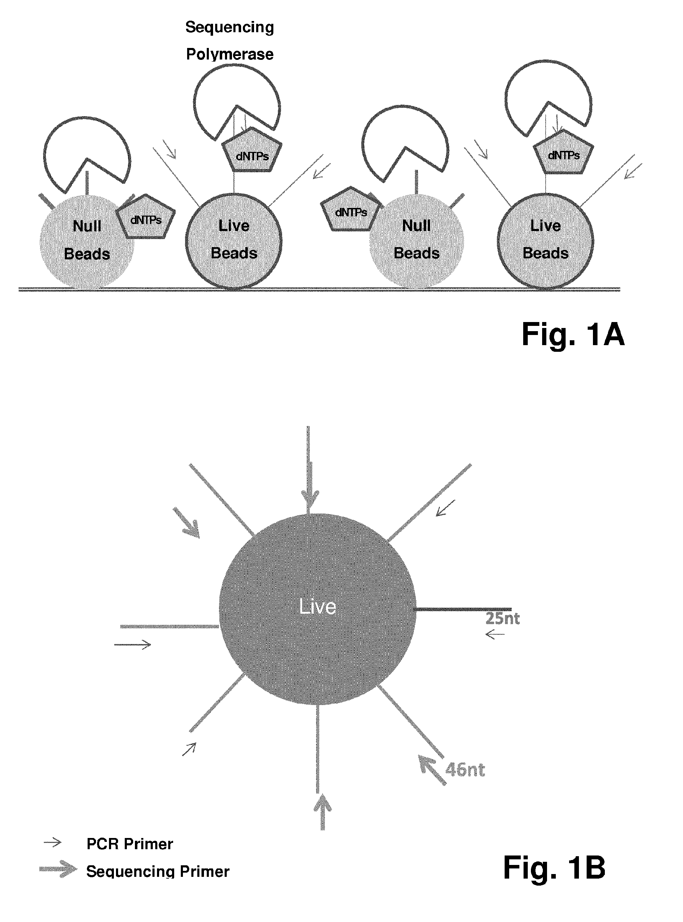

Two main hypotheses regarding the problem of suboptimal sequencing performances were addressed: One hypothesis is that primer-conjugated emulsion PCR where the bead-bound PCR primers are not extended ('Null Beads') can compete for sequencing reagents (such as sequencing polymerase, modified nucleotides) from the sequencing reaction in the flow cell and therefore limit sequencing efficiency (see schematic drawing in Figure 1A).

Another hypothesis is that the residual PCR primers from the emulsion PCR reaction can be carried over to the sequencing reaction despite extensive wash steps. The remaining PCR primer can bind to the single-stranded sequencing templates and compete with the sequencing primer for the binding sites on the adaptor sequences. Since the PCR primer is generally shorter than sequencing primer ( 25 nt for PCR primer vs. 46 nt for sequencing primer in this example), the hybridization of the PCR primer onto the sequencing template would lead to two different sequencing initiation sites that are extended with different nucleotides on the same template bead in each cycle (see schematic drawing in Figure 1 B). Both possible scenarios would lead to lower primer extension efficiency from the correct sequencing primer, the reduction of the fluorescence signals on the bead surface, high degree of phasing, and background fluorescence leading to poor base calling and may limit overall read-length.

With the method according to the invention and described herein, the sequencing efficiency issues for both hypotheses mentioned above can be eliminated: according to the invention, single-stranded nucleic acid molecules, e.g. primers, either conjugated on bead surface or non-specifically sticking to the bead are specifically eliminated by employing one or more single-strand-specific nucleases that can specifically remove single-stranded DNA without affecting double-stranded amplicons on the bead surface.

Exemplarily, Exonuclease I (Exol), a 3'-5' single-strand exonuclease, was used to treat emulsion PCR beads after breaking and washing steps and prior to denaturation and sequencing primer hybridization. Surprisingly, a significant improvement in sequencing performance in all criteria could be detected, i.e. in signal intensity, mapped reads, perfect reads, and error rate.

Briefly, 0.075pg/µl (final concentration) PhiX library with the GeneReader-specific adaptors (X adaptor and B adaptor; QIAGEN, Hilden, Germany) was mixed with 2X GeneReader emulsion PCR Mix (1 X final concentration, containing polymerase, dNTPs, and buffer; QIAGEN), Dynal MyOne C1 Streptavidin Beads (LIFE Technologies) conjugated with M13 primer that has 5' bisbiotin modification, PCR Primer B2 (6µM final concentration; QIAGEN) and 0.05µM of bisbiotin modified forward primer to make up the aqueous phase. The aqueous phase and the proprietary QIAGEN oil phase, MB5, were mixed on the GeneRead QiaCube Prototype to generate water:oil emulsions. The emulsions were transferred to a 96-well PCR plate and subjected to PCR cycling with the following program:

| | |
|---|---|
| Initial denaturation: | 2 min at 94°C |
| 60 cycles of: | 15 sec at 94°C |
| | 15 sec at 57°C |
| | 1 min 15 sec at 72°C |
| Final Extension: | 2 min at 72°C |

Primer sequences were as follows:
Forward Primer that is conjugated on beads: 
5'- BisBio/TTT TTT TTT TTT TTT TTT TT TTT TTT TTG TAA AAC GAC GGC
CAG T -3' (SEQ ID No. 1)
Bisbiotin modified forward primer:
   5-BisBio/TTTTTTTTTTGTAAAACGACGGCCAGT (SEQ ID No. 2)
Reverse Primer for emulsion PCR (B2):
   5' ACT TCA ATT TAC TAT GTA GCA AAG G 3' (SEQ ID No. 3)

Following PCR, the emulsions were broken and washed on the GeneRead QiaCube Prototype (QIAGEN) with automated protocol according to the manufacturer's instructions.

Beads recovered after the emulsion breaking/washing steps were then washed once with 1X Exol buffer (New England Biolabs) and split to two parts: one part (control) was resuspended in 200µl reaction mix containing 1X Exol buffer only (final concentration); another part (Exol-treated) was resuspended in 200µl reaction mix containing 1X Exol buffer (final concentration) and 200U Exonuclease I (20U/µl, NEB, part # M0293S). The bead suspension was then incubated on a heated shaker (Eppendorf) at 37°C for one hour with constant shaking, followed by heat-inactivation of the Exol at 80°C for 20 minutes.

The beads were then washed twice in 1 ml PBST (PBS with 0.05% Tween), denatured with NaOH/Tween solution (0.2N NaOH, 0.1% Tween) for 5 minutes at room temperature to generate single-stranded sequencing template on the bead surface, and hybridized with sequencing primer Seq46 (sequence: 5'ACT TCA ATT TAC TAT GTA GCA AAG GAT ACT CCG ACG CGG CCG CAG C 3' (SEQ ID No. 4)

The emulsion PCR beads, either control or Exol -treated, were then crosslinked to different flow cells and sequenced on GeneReader for 101 cycles.

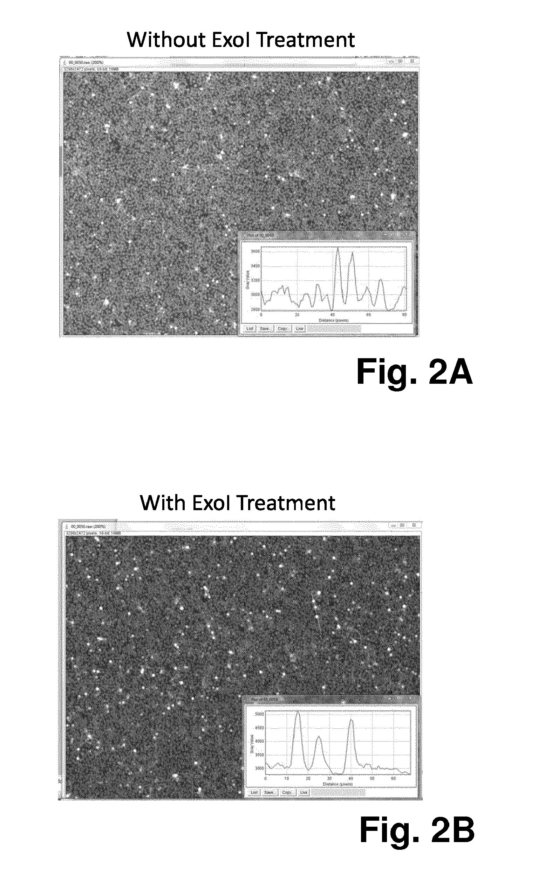

The sequencing images were analyzed with Image J software (http://imagej.nih.gov/ij/). As shown in Figure 2 where the T signals in cycle 1 were analyzed as an example, the signal intensity of the live emulsion PCR beads are significantly increased in the sample treated with Exol (Control: 3400-3800 counts; Exol-treated sample: 4000-5000 counts).

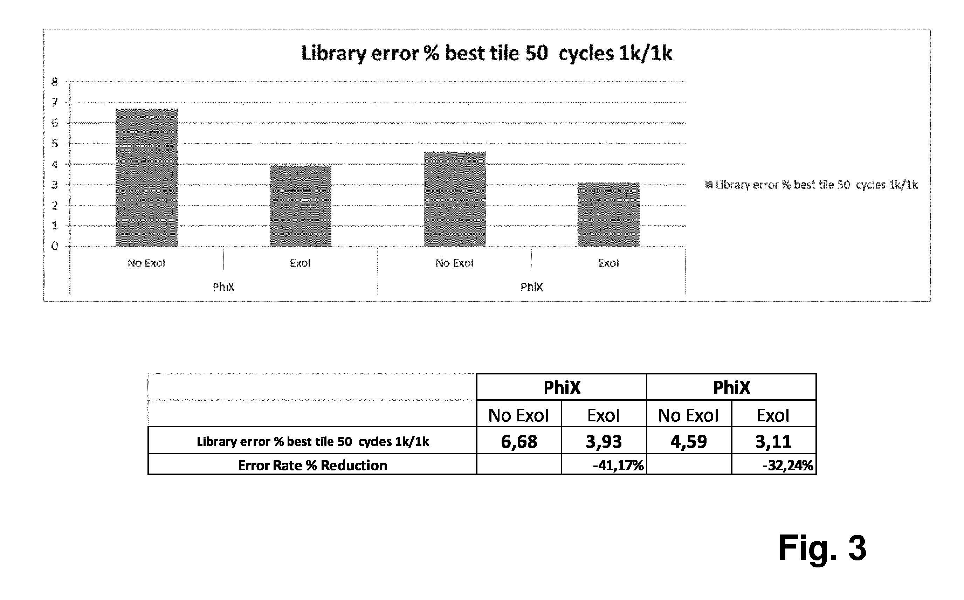

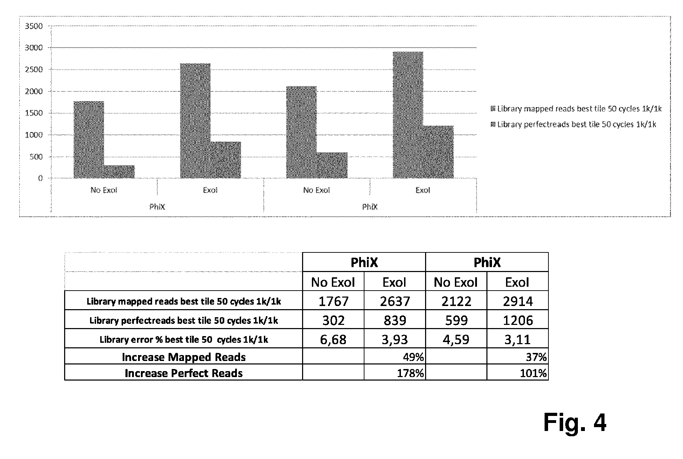

The beads at the center 1000pixeIX1000pixel area of each tile were analyzed with IBS Analyze software (QIAGEN) for mapped reads (reads mapped to PhiX genome with less than 3 errors in the first 28 nucleotides), perfect reads (reads perfectly mapped to the PhiX genome), and raw error rate at the read length of 50. The results of two independent experiments are shown in Figure 3 and Figure 4.

As shown in Figure 3 and Figure 4, Exol treatment significantly reduces sequencing error rate (32% and 41 % reduction), while increasing the numbers of mapped reads (37% and 49) and perfect reads (101% and 178%).

Taken together, the results clearly demonstrated that Exol treatment of the emulsion PCR beads significantly improves the sequencing performance of PCR products subsequently employed in downstream massively parallel sequencing processes.

Thus, the method according to the invention can be successfully employed in next generation sequencing workflows where a sequencing-by-synthesis process follows clonal amplification in order to remove free amplification primers that - after clonal amplification - will likely interfere with sequencing-by-synthesis process.

## Claims

1. Method for sequencing of target nucleic acids, the method comprising the steps of
(i) performing a clonal amplification step of target nucleic acids on a solid substrate to generate amplified amplification products coupled to the substrate in a reaction mixture,
(ii) treating the reaction mixture containing the amplified amplification products coupled to the solid substrate from step (i) with an enzyme exhibiting single-strand specific nuclease activity, and
(iii) subjecting the amplified amplification products obtained after treatment in step (ii) to a sequencing-by-synthesis process thereby sequencing the target nucleic acid.

2. The method of claim 1, **characterized in that** the clonal amplification step in step (i) is selected from at least one of the following: bridge amplification, emulsion polymerase chain reaction (PCR), rolling circle amplification, helicase depending amplification (HDA), recombinase polymerase amplification, Loop-mediated isothermal amplification (LAMP), Strand displacement amplification (SDA), Multiple displacement amplification (MDA), Nicking enzyme amplification reaction (NEAR), Multiple Annealing and Looping Based Amplification Cycles (a.k.a MALBAC), SPIA (Single Primer Isothermal Amplification), and WildFire Isothermal Amplification.

3. The method of any of claims 1 or 2, **characterized in that** in step (i) the clonal amplification step is an emulsion PCR to generate emulsion PCR beads having PCR products coupled thereto, that further in step (ii) the emulsion PCR beads from step (i) are treated with an enzyme exhibiting single-strand nuclease activity, and that the beads obtained after treatment in step (ii) are subjected to a sequencing-by-synthesis (SBS) process.

4. The method of any of claims 1 to 3, **characterized in that** the enzyme exhibiting single-strand-specific nuclease activity and employed in step (ii) is selected from a single-strand-specific exonuclease, or single-strand-specific endonuclease.

5. The method of any of claims 1 to 4, **characterized in that** the enzyme exhibiting single-strand-specific nuclease activity and employed in step (ii) is an enyzme exhibiting 3'->5' single-strand exonuclease activity.

6. The method of any of claims 1 to 4, **characterized in that** the enzyme exhibiting single-strand nuclease activity is selected from Exonuclease I (Exol) from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilage nuclease, and PfuExo I from *Pyrococcus furiosus.*

7. The method of any of claims 1 to 6, **characterized in that** step (ii) is performed for a time period of 10 min to 90 min.

8. The method of any of claims 2 to 7, **characterized in that** after step (i) and prior to step (ii) the emulsions are broken and washed.

9. The method of any of claims 1 to 8, **characterized in that** after step (ii) and prior to step (iii) the enzyme exhibiting single-strand nuclease activity is heat inactivated.

10. The method of any of claims 1 to 9, **characterized in that** after step (ii) and prior to step (iii) the amplified PCR products are denaturated to generate single-stranded sequencing templates on the substrate.

11. The method of any of claims 1 to 10, **characterized in that** prior to step (i) a library comprising the target nucleic acid is generated.

12. Use of an enzyme exhibiting single-strand nuclease activity in a sequencing process of a target nucleic acid, the sequencing process comprising a clonal amplification step and a sequencing-by-synthesis process.

13. The use of claim 12, **characterized in that** the enzyme exhibiting single-strand nuclease activity is selected from Exonuclease I (Exol) from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilage nuclease, and PfuExo I from *Pyrococcus furiosus.*

14. Kit for massively parallel sequencing processes, comprising (i) an enzyme exhibiting single-strand nuclease activity, and (ii) at least one or more of the following: a T4 ligase, Klenow fragment, amplification primer, one or more DNA polymerases, one or more adaptors, a solid surface, such as a bead or a set of beads, a substantially planar array, a well or wells in a plate, or an isolated surface, reagents for forming an emulsion buffer for one or more of the foregoing, and sequencing primer.

15. The Kit of claim 14, **characterized in that** the enzyme exhibiting single-strand nuclease activity is an exonuclease or endonuclease, and in particular selected from Exonuclease I (Exol) from *E. coli.,* Mung bean nuclease, S1 nuclease, nuclease P1, nucleases from *Alteromonas espejiana,* in particular Bal 31 endonuclease, nucleases from *Neurospora crassa,* in particular Neurospora endonuclease, nucleases from *Ustilago maydis,* in particular Ustilage nuclease, and PfuExo I from *Pyrococcus furiosus*
